Europäisches Patentamt

European Patent Office

Office européen des brevets

(11) Publication number: 0 363 219
A1

# EUROPEAN PATENT APPLICATION

(21) Application number: 89310259.0

(51) Int. Cl.5: A61B 5/00

(22) Date of filing: 06.10.89

(30) Priority: 07.10.88 US 254848

(43) Date of publication of application:
11.04.90 Bulletin 90/15

(84) Designated Contracting States:
AT BE CH DE ES FR GB GR IT LI LU NL SE

(71) Applicant: MEDEX, INC.
3637 Lacon Road
Hilliard Ohio 43026(US)

(72) Inventor: Barnes, Russell H.
1478 Berkshire Road
Columbus Ohio(US)
Inventor: Davis, Robert C.
1723 Grace Lane
Columbus Ohio 43220(US)
Inventor: Nicholson, Warren B.
1906 Queensbridge Drive
Worthington Ohio(US)

(74) Representative: Allen, Oliver John Richard et al
Lloyd Wise, Tregear & Co. Norman House
105-109 Strand
London, WC2R 0AE(GB)

(54) Oxygen sensor using phase shift.

(57) A phosphorescent sensor is subjected to a fluid containing oxygen. A light source modulated with a sine wave excites the sensor thereby producing an emitted light having an sine wave. The phase-shift between the exciting and emitted light sources is compared, the phase-shift being a measure of the pressure of the oxygen.

## Oxygen Sensor Using Phase-Shift

This invention relates to a method and apparatus for measuring partial pressure of oxygen, and in particular the invention relates to the in vivos measurement of the partial pressure of oxygen in the blood of humans.

Peterson et al. in Patent No. 4,476,870, discloses a method and apparatus for measuring oxygen in human blood. The patent describes a probe or sensor which is inserted in the oxygen-containing fluid (blood). The probe contains a fluorescent dye that is sensitive to oxygen quenching.

The dye is excited by a blue light and fluoresces. The fluorescence, a green light, is measured and the ratio of the intensity of the excitation light to the green light is a measure of the quenching effect of the oxygen in the blood. The problem with the Peterson method and other methods similar to it is that the calculation of blood pressure rests upon a determination of the absolute intensity of excitation and the intensity of the fluorescence. There are factors in the system that can cause variations of the intensity of fluorescence that are not easily controlled. The aging of the dye is one of them. The amount of dye is another. The quality of the matrix holding the dye is still another. Therefore, when measuring the level of intensity of fluorescence of the dye, the operator cannot be sure that the conditions have not varied from standard conditions under which the apparatus was calibrated.

It has been an objective of the present invention to provide a method and apparatus for measuring the partial pressure of oxygen in a fluid such as human blood and to eliminate the effects of variables that may cause variations in the intensity of the fluorescence of the excited dye.

In copending application Serial No. 07/155,964, filed February 16, 1988, there is disclosed an improved method and apparatus for measuring the partial pressure of oxygen in a fluid, particularly in a patient's blood. That method involves the measurement of the rate of decay of the intensity of the emitted light after a fluorophore sensor is pulsed with a laser excitation light. By measuring the rate of decay, the effects of extraneous factors as discussed above on the absolute intensity of the emitted light is eliminated. That system, however, requires a high intensity laser pulse. The apparatus for producing the laser beam is of itself extremely expensive.

The objective of the present invention has been to provide a method and apparatus for measuring partial pressure of oxygen which is significantly less expensive than prior apparatus and method requiring use of a laser excitation. The cost of the apparatus of the present invention is approximately one-fourth that of the prior apparatus requiring the use of laser.

The objective of the present invention is attained by exciting a phosphor with a monochromatic light that is sine wave modulated. The emitted light, of a different wavelength, will likewise be sine wave modulated. The phase difference between the two sine waves is a measure of the quenching effect of the oxygen present in the fluid and thus is a measure of the partial pressure of the oxygen.

Preferably, the system is calibrated to provide zero phase shift when the sensor is in a fluid that is 100% oxygen. As the percent of oxygen is diminished the phase-shift between the excitation and emitted light waves increases.

A phosphor is preferred as the sensor because of its slow decay time (milliseconds versus nanoseconds for fluorophores).

The light source can be an incandescent light passing through a filter to provide a substantially monochromatic light for excitation of the phosphor. Alternatively, a light-emitting diode LED may be employed for the excitation source.

The light source can be modulated with a wheel chopper having light passing through one or more openings in the wheel, or alternatively, the light source can be modulated using a conventional oscillator.

As the decay time of the phosphor is longer, it is possible to employ a lower frequency of modulation. The low frequency of modulation expands the list of suitable light sources permitting the use of an inexpensive light source. Similarly, inexpensive photodetectors can be employed for they have sufficient sensitivity for low frequency light signals even though the emitted light from the phosphor is relatively weak.

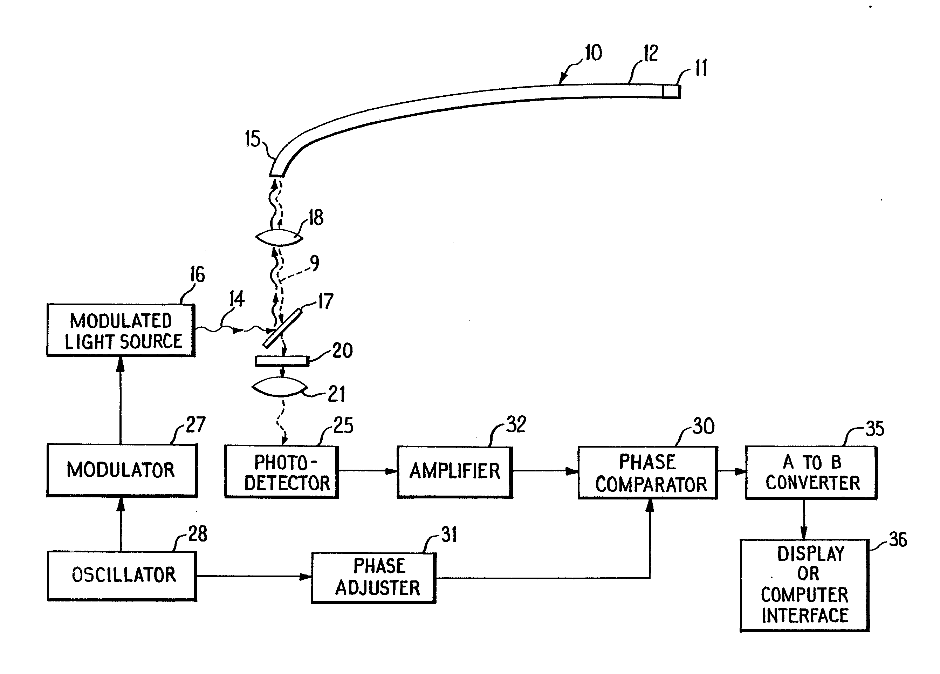

The several features and objectives of the present invention will become more readily apparent from the following detailed description taken in conjunction with the accompanying drawing which is a block diagram of the optical and electrical apparatus of the present invention.

Referring to the drawing, an optical fiber 10 has a sensor 11 mounted on its free end 12. The sensor is a phosphor that is immobilized in a gas-permeable support material such as silicon rubber. Europium or Erythrosin-B are suitable phosphors for use in the method and apparatus of the present invention.

The other end of the optical fiber 15 is connected to a modulated light source 16. The light source 16 is directed to a beam splitter 17 which reflects at least 95% of the light of the excitation wavelength. A suitable excitation wavelength for Erythrosin-B is about 500 nanometers. The excitation light is focused by a lens 18 and directed into the optical fiber 10 to excite the phosphor in the sensor 11.

The excitation of the phosphor will cause a light of a wavelength of about 650 nanometers to be emitted. That light will pass down the optical fiber and be directed through the lens 18 onto the beam splitter 17. The beam splitter 17 is adapted to transmit at least 80% of the light of the emission wavelength.

The emitted light passes through a filter 20 to eliminate extraneous wavelengths and a lens 21 focuses the light onto a photodetector 25.

The light source 16 is modulated by a modulator 27, the modulator 27 being driven by an oscillator 28 at a preselected sine wave frequency. A frequency of 100 Hz is an example of a suitable modulation frequency. Just as the light source is sine wave modulated, so the emitted wave 19 is sine wave modulated.

A phase comparator 30 is provided to provide a signal proportional to the phase difference between the excitation and emission sine waves. The excitation sine wave is connected to the phase converter through a phase adjuster 31. The emission light is connected to the phase comparator through an amplifier 32.

The phase comparator produces an analog voltage proportional to the degree of phase-shift between the excitation and emission signals. That analog output is connected to an analog-to-digital converter 35. The digital signal from the converter 35 is connected to a display or computer interface 36 where the partial pressure of the blood is presented in real time.

Before being put into use, the apparatus is factory-adjusted using the phase adjuster 31. To make the adjustment, the sensor is put in an atmosphere of 100% oxygen and the adjustment is made to zero phase-shift. As the percentage of oxygen is decreased, the phase shift will increase.

In operation, the sensor is inserted into the patient's blood vessel system where the sensor 11 is subjected to blood. The oxygen contacts the phosphor in the sensor. The sensor is excited by the sine wave modulated light source producing an excitation signal 14. The excitation signal impinges on the phosphor and excites it to produce an emission wave form 19 which is a sine wave of the same frequency as the excitation signal 14. That signal will be shifted in phase with respect to the excitation signal by an amount that is inversely proportional to the partial pressure of the oxygen in the blood.

The two signals are fed to the phase comparator to produce an output voltage inversely proportional to the phase-shift. That signal, via an analog-to-digital converter is displayed in real time and in terms of partial oxygen pressure such as millimeters of mercury.

From the above disclosure of the general principles of the present invention and the preceding detailed description of a preferred embodiment, those skilled in the art will readily comprehend the various modifications to which the present invention is susceptible. Therefore, we desire to be limited only by the scope of the following claims and equivalents thereof.

## Claims

1. Apparatus for measuring partial pressure of oxygen in a fluid comprising,
a sensor which, when excited at a first wavelength, produces an emission at a second wavelength,
means for exciting said sensor with a sine wave modulated light source to produce an emitted light,
meand for comparing the phase shift of said emitted light with respect to the references sine wave of said excitation light, the amount of phase-shift forming a measure of partial pressure of $O_2$.

2. Apparatus for measuring partial pressure of oxygen in blood comprising,
an optical fiber having a sensor on one end thereof,
a substantially monochromatic light source,
means connected to said light source for modulating said light source with a sine wave,
means for focusing said light source on the other end of said fiber, whereby said light source will excite said sensor to emit an emitted light modulated with said sine wave,
a photodetector positioned to receive said emitted light for converting said light to a sine wave voltage,
a phase comparator,
means connecting said modulating means and said photodetector to said phase comparator to compare the phase shift of said emitted light to the modulating wave,
and display means connected to said phase comparator for receiving the output of said phase comparator and displaying said output as a partial oxygen pressure.

3. Apparatus as in claim 2 further comprising, phase adjust means for providing a zero phase-shift when said sensor detects 100% oxygen.

4. Apparatus as in claim 1 in which said sensor is a phosphor.

5. Apparatus as in claim 1 in which said sensor is a phosphor and is excited at a frequency of about 1000 Hz.

6. The method of measuring the pressure of oxygen in a fluid using a sensor that emits light of one frequency when excited by light of another frequency and whose emission is quenched by the presence of oxygen comprising the steps of,
placing said sensor in said fluid,
modulating as light source with a sine wave, exciting said sensor with said modulated light source, thereby generating an emitted light modulated by said sine wave,
measuring the phase difference between the sine wave of said source light and said emitted light,
and converting said phase difference to oxygen pressure.

EP 0 363 219 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y | EP-A-0 252 578 (J.E. SCHULZE) * Page 2, lines 4-6; page 3, lines 29-31; page 5, lines 15-51; page 8, line 25 - page 9, line 20; figures 1-5 * | 1,6 | A 61 B 5/00 |
| A | | 4 | |
| Y | US-A-4 200 801 (D.D. SCHURESKO) * Abstract; column 1, lines 6-13; column 2, lines 15-34; column 2, lines 11-24; column 4, lines 9-60; column 5, lines 10-58; column 6, lines 29-33; column 6, line 65 - column 7, line 48; figures 2-4 * | 1,6 | |
| A | | 2,3,5 | |
| A | US-A-4 608 990 (V.B. ELINGS) * The whole document * | 1,2,6 | |
| A | DD-A- 217 633 (K. BERNDT) * The whole document * | 1,2 | TECHNICAL FIELDS SEARCHED (Int. Cl.4) |
| A | THE REVIEW OF SCIENTIFIC INSTRUMENTS, vol. 36, no. 8, August 1965, pages 1214-1226, New York, US; A. MÜLLER et al.: "High performance phase fluorometer constructed from commercial subunits" | 1,2 | A 61 B G 01 N |

The present search report has been drawn up for all claims

| Place of search | Date of completion of the search | Examiner |
|---|---|---|
| THE HAGUE | 20-12-1989 | FERRIGNO, A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)